(19) Europäisches Patentamt / European Patent Office / Office européen des brevets

(11) **EP 2 902 102 B1**

(12) **EUROPEAN PATENT SPECIFICATION**

(45) Date of publication and mention
of the grant of the patent:
**06.11.2019 Bulletin 2019/45**

(21) Application number: **13841865.2**

(22) Date of filing: **27.09.2013**

(51) Int Cl.:
*B01J 13/00* (2006.01)    *A23D 7/00* (2006.01)
*A61K 8/06* (2006.01)    *A61K 8/31* (2006.01)
*A61K 8/36* (2006.01)    *A61K 8/37* (2006.01)
*A61K 8/86* (2006.01)    *A61Q 5/02* (2006.01)
*A61Q 11/00* (2006.01)    *A61Q 15/00* (2006.01)
*A61Q 19/00* (2006.01)

(86) International application number:
**PCT/JP2013/076418**

(87) International publication number:
**WO 2014/051115 (03.04.2014 Gazette 2014/14)**

(54) **CAROTENOID-CONTAINING OIL-IN-WATER EMULSION COMPOSITION**

CAROTINOIDHALTIGE ÖL-IN-WASSER-EMULSIONSZUSAMMENSETZUNG

COMPOSITION D'ÉMULSION D'HUILE DANS L'EAU CONTENANT UN CAROTÉNOÏDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.09.2012 JP 2012217830**

(43) Date of publication of application:
**05.08.2015 Bulletin 2015/32**

(73) Proprietor: **FUJIFILM Corporation
Tokyo 106-8620 (JP)**

(72) Inventors:
• **KITAOKA, Hiroyuki
Ashigarakami-gun
Kanagawa 258-8577 (JP)**
• **MORI, Mikinaga
Ashigarakami-gun
Kanagawa 258-8577 (JP)**

(74) Representative: **Klunker IP
Patentanwälte PartG mbB
Destouchesstraße 68
80796 München (DE)**

(56) References cited:
**EP-A1- 1 864 578        EP-A1- 2 380 552
WO-A1-2009/075383      WO-A1-2010/038816
JP-A- 2003 292 428      JP-A- 2003 306 423
JP-A- 2010 083 801      JP-A- 2010 155 815
US-A1- 2009 148 343**

**Description**

**Technical Field**

The present invention relates to a carotenoid-containing oil-in-water emulsion composition.

**Background Art**

In recent years, attention has been paid to the high functionality of carotenoids, and various compositions containing carotenoid have been suggested. In general, carotenoids are widely known as materials that are sparingly soluble in water, and various technologies have been proposed from the viewpoints of solubility and dispersibility of carotenoids.

Specific examples of compositions containing carotenoids known so far include a carotenoid-containing emulsion composition that contains at least one water-soluble emulsifier in an aqueous phase, and contains tocopherol and lecithin in an oil phase (see Japanese Patent Application Laid-Open (JP-A) No. 2008-13751); a carotenoid-based food pigment-solubilized liquid preparation for food, in which a composition of a carotenoid-based food pigment and a polyglycerin fatty acid ester is micronized, and when the absorbance at the maximum absorption wavelength in the visible region is 1, the transmittance at 660 nm is 99% or more (see JP-ANo. H10-120933); and a carotenoid-containing composition in which an oil phase obtained by dissolving carotenoids in oils and fats is emulsified in an aqueous phase containing a polyhydric alcohol in the presence of a polyglycerin fatty acid ester and lecithin, and the average particle diameter of the oil phase is 100 nm or less (see JP-A No. H09-157159). Furthermore, JP-ANo. 2011-241177 describes a carotenoid-containing composition obtained by heating a mixed liquid of a carotenoid component including crystalline carotenoid and an oil phase component containing a specific (poly)glycerin fatty acid ester, under particular temperature conditions.

JP 2010-155815 A discloses an astaxanthin-containing dispersion containing stably dispersed particles having a small particle diameter and exhibiting excellent stability with time. The astaxanthin-containing dispersion contains (1) dispersion particles containing astaxanthin and ceramides, dispersed in an aqueous phase as an oil phase component and having a volume-average particle diameter of $\geq 1$ nm and $\leq 100$ nm and (2) at least one kind of fatty acid component selected from (a) a 12-20 C fatty acid and (b) a fatty acid salt, provided that the amount of surfactants other than the fatty acid component is 0 or $\leq 0.1$ times mass based on the total mass of the oil component containing the ceramides and astaxanthin, and the pH of the dispersion is $\geq 6$ and $\leq 8$.

**SUMMARY OF INVENTION**

**Technical Problem**

Among carotenoids, lycopene, in particular, is a compound which is prone to oxidative decomposition and has inferior stability, and lycopene may be easily oxidatively decomposed due to changes in the environmental temperature or long-term storage.

Furthermore, when an oil-in-water emulsion composition is stored for a long time, addition of an antiseptic agent or a heat sterilization treatment may be necessary as a countermeasure against putrefaction. Further, substances that are prone to oxidative decomposition, such as lycopene, may undergo accelerated oxidative decomposition as a result of addition of an antiseptic agent. Therefore, in a case in which an oil-in-water emulsion composition containing a carotenoid such as lycopene is stored for a long time, it is preferable to perform a heat sterilization treatment rather than adding an antiseptic agent.

However, an oil-in-water emulsion composition containing an oil-in-water emulsion composition containing a carotenoid such as lycopene is problematic in that, particularly in a case in which the particle diameter of the emulsion particles is small, emulsion destruction such as coarsening of the emulsion particle diameter by high temperature heating is likely to occur. Such coarsening of the emulsion particle diameter is undesirable, particularly when transparency is required in an emulsion composition.

The invention was made in view of such circumstances, and it is an object of the invention to provide a carotenoid-containing oil-in-water emulsion composition which stably maintains an emulsified state with respect to heating treatment and has excellent storage stability.

**Solution to Problem**

Means for solving the problems described above are as follows.

[1] A carotenoid-containing oil-in-water emulsion composition, including: a carotenoid; a higher fatty acid having a total carbon number of 12 or more in an amount of from 0.001 times to 2 times by mass ratio with respect to the carotenoid; and a surfactant other than the higher fatty acid having a total carbon number of 12 or more, wherein an average particle diameter of emulsion particles is 120 nm or less, wherein a content of the surfactant other than the higher fatty acid having a total carbon number of 12 or more is from 6% by mass to 15% by mass with respect to the total mass of the emulsion composition, and wherein the surfactant is a polyglycerin fatty acid ester having an HLB value of from 10 to 20 and having 6 or more glycerin units.

[2] The carotenoid-containing oil-in-water emulsion composition according to [1], wherein the carotenoid is lycopene.

[3] The carotenoid-containing oil-in-water emulsion composition according to [1] or [2], including the higher fatty acid having a total carbon number of 12 or more in an amount of from 0.01 times to 0.5 times by mass ratio with respect to the carotenoid.

[4] The carotenoid-containing oil-in-water emulsion composition according to any one of [1] to [3], wherein the average particle diameter of the emulsion particles is 100 nm or less.

[5] The carotenoid-containing oil-in-water emulsion composition according to any one of [1] to [4], wherein the higher fatty acid having a total carbon number of 12 or more is a higher fatty acid having a total carbon number of from 18 to 22.

[6] The carotenoid-containing oil-in-water emulsion composition according to any one of [1] to [5], wherein a total content of an antiseptic agent and a preservative is 0.001% by mass or less with respect to the total mass of the emulsion composition.

[7] A method of producing an emulsion composition, the method including: preparing the carotenoid-containing oil-in-water emulsion composition according to any one of [1] to [6], subsequently heating the emulsion composition to 70°C or higher immediately before or after filling a storage container with the emulsion composition, and sealing the storage container that has been filled with the heated emulsion composition.

**Advantageous Effects of Invention**

According to the invention, there is provided a carotenoid-containing oil-in-water emulsion composition which stably maintains an emulsified state with respect to a heating treatment and has excellent storage stability.

**DESCRIPTION OF EMBODIMENTS**

Hereinafter, the invention will be described in detail.

The carotenoid-containing oil-in-water emulsion composition of the invention (hereinafter, may be appropriately referred to as "emulsion composition of the invention") is an oil-in-water emulsion composition including: a carotenoid, a higher fatty acid having a total carbon number of 12 or more in an amount of from 0.001 times to 2 times by mass ratio with respect to the carotenoid, a surfactant other than a higher fatty acid having a total carbon number of 12 or more; and an average particle diameter of emulsion particles is 120 nm or less, wherein a content of the surfactant other than the higher fatty acid having a total carbon number of 12 or more is from 6% by mass to 15% by mass with respect to the total mass of the emulsion composition, and wherein the surfactant is a polyglycerin fatty acid ester having an HLB value of from 10 to 20 and having 6 or more glycerin units.

The emulsion composition of the invention having the configuration described above maintains excellent emulsification stability with respect to heating treatment and excellent storage stability over a long time period.

That is, the emulsion composition of the invention can maintain the particle diameter of emulsion particles at a desired particle diameter even in a case in which a heating treatment (about 75°C to 85°C) is carried out. For this reason, the emulsion composition of the invention can be subjected to an antiseptic treatment such as a high temperature sterilization treatment without damaging the emulsified state.

Furthermore, according to the invention, the particle diameter of emulsion particles is stably maintained at a fine particle diameter (preferably, an average particle diameter of 120 nm or less). For this reason, the emulsion composition of the invention is particularly suitable as a transparent emulsion composition having antiseptic properties.

The emulsion composition of the invention is an oil-in-water emulsion composition, and can be obtained by mixing and emulsification of an aqueous phase composition composed of aqueous phase components and an oil phase composition composed of oil phase components.

Furthermore, the emulsion particles in the emulsion composition of the invention exist as an oil phase (dispersed phase) in the emulsion composition of the invention, which is an oil-in-water type.

In the invention, any numerical range expressed herein using "to" refers to a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

Furthermore, in the invention, the "stable maintenance of an emulsified state with respect to heating treatment" means that fluctuations in the particle diameter of emulsion particles are suppressed even in a case in which the emulsion composition is heated (about 75°C to 85°C).

In the invention, "storage stability" means that decomposition of a carotenoid such as lycopene that is included in the composition is suppressed and fluctuations in the particle diameter of emulsion particles are suppressed, even in a case in which the emulsion composition is aged.

In the invention, the "dissolution temperature of a carotenoid" means the lowest temperature at which, when a carotenoid crystalline body or a composition including a carotenoid crystalline body is maintained for one minute at that temperature, the carotenoid crystalline body completely dissolves.

Hereinbelow, the various constituent elements of the invention will be explained in more detail.

### <Carotenoid>

The emulsion composition of the invention includes a carotenoid.

It is preferable that a carotenoid is included as one of the constituent components of the emulsion particles in the emulsion composition of the invention.

Carotenoids are terpenoid-based pigments having a color ranging from yellow to red, and examples thereof include carotenoids derived from plants, algae, and bacteria. Furthermore, carotenoids are not limited to naturally occurring ones, and any carotenoid substance obtainable by a conventional method may be used.

Examples of the carotenoid in the invention include lycopene, α-carotene, β-carotene, γ-carotene, δ-carotene, actinioerythrol, bixin, canthaxanthin, capsorubin, β-8'-apo-carotenal (apocarotenal), β-12'-apo-carotenal, xanthophylls (for example, astaxanthin, fucoxanthin, lutein, zeaxanthin, capsanthin, β-cryptoxanthin, and violaxanthin and the like), and hydroxyl or carboxyl derivatives thereof. These carotenoids may be used singly, or in combination of two or more kinds thereof.

The carotenoid in the invention is a crystalline carotenoid. Here, the "crystalline carotenoid" is not intended to mean a certain particular carotenoid, but means any carotenoid that can exist as a crystalline body at any one temperature in a temperature range of from -5°C to 35°C, owing to various factors such as the method of production thereof, treatment, and storage, when the carotenoid exists in the form of an oil, a paste or the like containing carotenoid. Particularly, lycopene, β-carotene, δ-carotene, zeaxanthin, lutein, astaxanthin, and the like that will be described below are carotenoids whose crystalline bodies can easily exist.

It is preferable that the emulsion composition of the invention includes lycopene among the carotenoids.

Lycopene is a carotenoid represented by the chemical formula: $C_{40}H_{56}$ (molecular weight 536.87), and is a red pigment which belongs to carotenes, which is a class of carotenoids, and exhibits an absorption maximum at 474 nm (acetone).

Lycopene is known to be excellent in an antioxidant effect, a skin whitening effect and the like, and addition thereof to the raw materials of foods, cosmetics and pharmaceuticals, and processed products thereof has been hitherto desired, investigated, and implemented.

Lycopene also has cis- and trans-isomers of the conjugated double bond at the center of the molecule, and examples thereof include an all-trans form, a 9-cis form, and a 13-cis form. According to the invention, any of these lycopene forms may be used.

Lycopene may also be used as a lycopene-containing oil or a lycopene-containing paste, which has been separated and extracted from a natural product containing lycopene, in the preparation of the emulsion composition of the invention.

Lycopene is contained in natural products such as tomato, persimmon, watermelon, and pink grapefruit, in nature. The lycopene-containing oil may be an oil that has been separated and extracted from these natural products.

Regarding the form of a product containing lycopene, there are known four kinds such as an oil type, an emulsion type, a paste type, and a powder type.

Furthermore, the lycopene used in the invention may be a product obtained by appropriately purifying an extract from a natural product as necessary. Furthermore, the lycopene used in the invention may also be a synthetic product.

One of particularly preferred forms of lycopene in the invention may be an oil-soluble extract extracted from tomato pulp. An oil-soluble extract extracted from tomato pulp is particularly preferable from the viewpoints of stability, product quality, and productivity of a composition including the relevant oil-soluble extract.

Here, an oil-soluble extract extracted from tomato pulp means an extract extracted from a pulp-like solid obtained by centrifuging a crushed product obtained by crushing tomatoes, using an oily solvent.

Regarding lycopene as an oil-soluble extract, tomato extracts that are widely sold in the market as lycopene-containing oils or lycopene-containing pastes can be used. Examples of tomato extracts that are commercially available include LYC-O-MATO 15% and LYC-O-MATO 6% available from Sunbright Co., Ltd.; and LYCOPENE 18 available from Kyowa Hakko Kogyo Bio Co., Ltd.

It is preferable that the carotenoid in the invention is included in the emulsion composition of the invention in a non-crystalline state. When a crystalline carotenoid is in a non-crystalline state, it is easier to adjust the average particle diameter of the emulsion particles to 120 nm or less, and absorbability of the carotenoid in the body can be further increased.

The question of whether or not a crystalline carotenoid including lycopene is non-crystalline, may be confirmed by using a known means for detecting a crystal structure. For example, the fact of being a crystalline carotenoid may be confirmed by a conventional method, and differential scanning calorimetry (DSC), observation by polarization microscopy, X-ray diffraction, and the like can be utilized. When it is found that detection of a crystalline body cannot be confirmed by these known technologies, the carotenoid can be regarded as being non-crystalline. In particular, in this invention, it is preferable to confirm that the carotenoid is non-crystalline based on the presence of a DSC endotherm peak.

In regard to whether or not a crystalline carotenoid is in a non-crystalline state, it is preferable to confirm whether or not at least one of the following is satisfied: that the endothermic and exothermic temperatures are determined, using a DSC Q2000 (TA Instruments Japan, Inc.), in one cycle of temperature increase-temperature decrease (15°C/min) in a temperature range of from 30°C to 200°C, in a state that moisture has been eliminated through freeze-drying, and the presence of a recognizable endotherm peak is not recognized; and that when an emulsion composition having a content of a crystalline carotenoid of 0.1% by mass is observed by polarization microscopic observation, there are 1000 or fewer recognizable crystalline bodies in a viewing field that measures 1 cm $\times$ 1 cm.

Furthermore, regarding the carotenoid component such as lycopene included in the invention, it is preferable, from the viewpoint of dynamic absorbability, that at least from 50% by mass to 100% by mass of the crystalline carotenoid is non-crystalline, it is more preferable that from 90% by mass to 100% by mass is non-crystalline, and it is still more preferable that from 95% by mass to 100% by mass is non-crystalline.

The question of whether or not the carotenoid component includes at least 50% by mass of a crystalline carotenoid that is non-crystalline can be confirmed, for example, by comparing the amount of heat absorption of an endotherm peak originating from carotenoid crystals in the composition of the invention measured by differential scanning calorimetry (DSC) with the amount of heat absorption of an endotherm peak of a carotenoid crystal standard product.

Further, this can also be confirmed by comparing the spectrum of the emulsion composition of the invention in X-ray diffraction with the spectrum of a carotenoid crystal standard product.

The content ratio of a crystalline carotenoid including lycopene, which is non-crystalline, can be calculated from a result obtained using a commercially available carotenoid reagent as a crystalline body, and determining the content ratio from the DSC peak area or XRD (X-ray diffraction) based on the carotenoid reagent being taken as 100%. Examples of the commercially available product of a carotenoid reagent as a crystalline body include the reagents for biochemical applications available from Wako Pure Chemical Industries, Ltd.

The crystalline carotenoid including lycopene may constitute the carotenoid component as a simple substance, or may constitute the carotenoid component together with the oil component (oil) used to extract the carotenoid from a natural product.

The carotenoid component may include naturally occurring non-crystalline carotenoid (amorphous carotenoid), in addition to the crystalline carotenoid.

The content of the carotenoid in the emulsion composition of the invention is preferably from 0.1% by mass to 5% by mass, more preferably from 0.2% by mass to 4% by mass, and still more preferably from 0.3% by mass to 3% by mass, with respect to the total mass of the solid content (all components excluding water) in the emulsion composition. When the content is in this range, it can be expected to obtain further effects provided by the carotenoid.

**<Higher fatty acid having total carbon number of 12 or more>**

The emulsion composition of the invention includes a higher fatty acid having a total carbon number of 12 or more (hereinafter, may be simply referred to as "higher fatty acid"). The higher fatty acid in the emulsion composition of the invention is preferably included as one of the constituent components of the emulsion particles.

The higher fatty acid in the invention may be any of a straight-chained fatty acid or a branched fatty acid, and may be a saturated fatty acid or an unsaturated fatty acid.

The higher fatty acid according to the invention is a higher fatty acid having a total carbon number of 12 or more, and from the viewpoint of stably maintaining an emulsified state with respect to heating treatment, a higher fatty acid having a total carbon number of from 12 to 22 is preferred, while a higher fatty acid having a total carbon number of from 18 to 22 is more preferred.

Specific examples of the higher fatty acid in the invention include lauric acid, myristic acid, palmitic acid, stearic acid, isostearic acid, oleic acid, linoleic acid, $\alpha$-linolenic acid, $\gamma$-linolenic acid, arachidic acid, arachidonic acid, behenic acid, docosahexaenoic acid, eicosapentaenoic acid, erucic acid, and the like.

The higher fatty acid that is included in the emulsion composition of the invention may be used singly, or in combination of two or more kinds.

The content of the higher fatty acid in the emulsion composition of the invention is from 0.001 times to 2 times, and preferably from 0.01 times to 0.5 times, with respect to the carotenoid by mass ratio.

In the invention, when the content of the higher fatty acid with respect to the carotenoid is adjusted to the range described above, and a specific surfactant that will be described below is used in combination therewith, an emulsion composition having emulsion particles having a fine particle diameter (preferably, the average particle diameter is 120 nm or less) can be obtained. Furthermore, even if the emulsion composition is subjected to a treatment such as heating, enlargement of the particle diameter is effectively suppressed, and decomposition of the carotenoid such as lycopene over time can also be suppressed.

**<Surfactant>**

The emulsion composition of the invention includes a surfactant. The surfactant may be used singly, or in combination of two or more kinds. However, the surfactant is not intended to include higher fatty acids having a total carbon number of 12 or more.

The surfactant according to the invention can function as an emulsifier, and for the emulsion composition of the invention which is of oil-in-water type, it is preferable to use the surfactant as one component of the aqueous phase composition.

The surfactant may be any of an anionic surfactant, a cationic surfactant, an amphoteric surfactant, or a nonionic surfactant.

Further, from the viewpoint of emulsifying power, the surfactant is one having an HLB value of 10 or more, and preferably one having an HLB value of 12 or more. If the HLB value is too low, the emulsifying power may be sufficient. In addition, from the viewpoint of a defoaming effect, an emulsifier having an HLB value of from 5 or more but less than 10 may be used in combination.

Here, the HLB is the hydrophilicity-hydrophobicity balance that is conventionally used in the field of surfactants, and calculation formulas that are conventionally used, for example, Kawakami's formula, can be used. The Kawakami's formula is shown below.

$$HLB = 7 + 11.7\log(M_w/M_o)$$

Here, $M_w$ represents the molecular weight of a hydrophilic group, and $M_o$ represents the molecular weight of a hydrophobic group.

Furthermore, the values of HLB described in catalogues and the like may also be used.

As can be seen from the formula described above, an emulsifier having an arbitrary HLB value can be obtained by using the additivity of the HLB.

From the viewpoints of micronization of emulsion particles and suppression of decomposition of lycopene, the content of the surfactant in the emulsion composition is from 6% by mass to 15% by mass, with respect to the total mass of the emulsion composition.

Furthermore, regarding the total mass of the surfactant, the surfactant can be used in an amount range of from 0.1 times to 10 times with respect to the total mass of the oily components including the carotenoid, and from the viewpoints of micronization of emulsion particles and suppression of foaming, the total mass is preferably from 0.5 times to 8 times, and particularly preferably 0.8 times to 5 times. When the total mass is in this range, emulsification stability of the emulsion composition can be made satisfactory.

Among the surfactants, a nonionic surfactant is preferred because a nonionic surfactant is less irritant and has less adverse effect on the environment. The surfactant used in the present invention is a polyglycerin fatty acid ester. Examples of a nonionic surfactant not falling under the scope of the present invention include a sucrose fatty acid ester, an organic acid monoglyceride, a propylene glycol fatty acid ester, a polyglycerin condensed ricinoleic acid ester, a sorbitan fatty acid ester, a polyoxyethylene sorbitan fatty acid ester, or a combination.

Among these nonionic surfactants, a polyglycerin fatty acid ester is used from the viewpoint of obtaining stable and fine emulsion particles.

The sucrose fatty acid ester is such that, from the viewpoint of stability of the dispersed particles in the composition, the carbon number of the fatty acid that constitutes the sucrose fatty acid ester is preferably from 12 to 20, and more preferably from 14 to 16.

Preferred examples of the sucrose fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester. Among these, sucrose monooleic acid ester, sucrose monostearic acid ester, sucrose monopalmitic acid ester, sucrose monomyristic acid ester, and sucrose monolauric acid ester are more preferred.

These sucrose fatty acid esters can be used singly or in combination.

The polyglycerin fatty acid ester that is included as a surfactant is an ester of a polyglycerin having an average degree of polymerization of 6 or more (preferably from 6 to 15, and more preferably from 8 to 10) and a fatty acid having preferably a carbon number of from 8 to 18. Examples of the fatty acid having a carbon number of from 8 to 18 include caprylic acid, capric acid, lauric acid, myristic acid, palmitic acid, stearic acid, oleic acid, and linoleic acid.

Preferred examples of the polyglycerin fatty acid ester that is included as a surfactant include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, and decaglycerin monolauric acid ester.

Among these, more preferred are decaglycerin monooleic acid ester (HLB = 12), decaglycerin monostearic acid ester (HLB = 12), decaglycerin monopalmitic acid ester (HLB = 13), decaglycerin monomyristic acid ester (HLB = 14), and decaglycerin monolauric acid ester (HLB = 16) and the like.

In the invention, these polyglycerin fatty acid esters that are included as surfactants can be used singly or in combination.

As the sorbitan fatty acid ester, such a compound having a carbon number of the fatty acid of 8 or more is preferred, and a compound having a carbon number of the fatty acid of 12 or more is more preferred. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, sorbitan trioleate, and the like.

These sorbitan fatty acid esters can be used singly or in combination.

As the polyoxyethylene sorbitan fatty acid ester, such a compound having a carbon number of the fatty acid of 8 or more is preferred, and a compound having a carbon number of the fatty acid of 12 or more is more preferred. The length of ethylene oxides (number of added moles) of the polyoxyethylene is preferably from 2 to 100, and more preferably from 4 to 50.

Preferred examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, polyoxyethylene sorbitan trioleate, and the like.

These polyoxyethylene sorbitan fatty acid esters can be used singly or in combination.

Among the surfactants described above, the surfactant for the invention is a polyglycerin fatty acid ester having an HLB value of from 10 to 20 and containing 6 or more glycerin units, from the viewpoint of obtaining stable and fine emulsion particles.

**<Other emulsifying components>**

In addition, this invention may also include a phospholipid such as lecithin as an emulsifier.

The phospholipid that can be used in the invention is a compound which contains a glycerin skeleton, a fatty acid residue, and a phosphoric acid residue as essential constituent components, and has a base, a polyhydric alcohol or the like bonded thereto. The phospholipid is also called lecithin. Since a phospholipid has a hydrophilic group and a hydrophobic group in the molecule, phospholipids have been widely used hitherto as emulsifiers in the fields of foods, cosmetics, and pharmaceuticals.

Industrially, a product having a lecithin purity of 60% or more is utilized as lecithin, and that can also be utilized in this invention. From the viewpoints of the formation of fine oil droplet particle diameter and the stability of functional oily components, preferred lecithin is what is generally referred to as high purity lecithin, and this is a product having a lecithin purity of 80% or more, and more preferably 90% or more.

Examples of the phospholipid include various conventionally known phospholipids that are extracted and separated from living organisms such as plants, animals, or microorganisms.

Specific examples of such phospholipids include various lecithins derived from plants such as soybean, corn, peanut, rapeseed, and wheat; animals such as egg yolk and beef; and microorganisms such as Escherichia coli.

Examples of such lecithin include, expressed in their compound names, glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); and sphingolecithins such as sphingomyelin.

Also, according to the invention, hydrogenated lecithin, enzymatically degraded lecithin, enzymatically degraded hydrogenated lecithin, hydroxylecithin, and the like can also be used in addition to the high purity lecithin. These lecithins that can be used in the invention can be used singly, or in mixture of plural kinds thereof.

### <Other (poly)glycerin fatty acid esters>

The emulsion composition of the invention may include, as one of the constituent components contained in the emulsion particles, a (poly)glycerin fatty acid ester described below (hereinafter, appropriately referred to as "other (poly)glycerin fatty acid ester"). The other (poly)glycerin fatty acid ester is not included in the surfactants according to the invention described above.

The other (poly)glycerin fatty acid ester is a (poly)glycerin fatty acid ester having a number of glycerin unit(s) of from 1 to 5, a number of fatty acid unit(s) of from 1 to 6, and at least one hydroxyl group of a glycerin unit.

In a co-dissolution product of such a specific (poly)glycerin fatty acid ester and a crystalline carotenoid such as lycopene, recrystallization of the crystalline carotenoid is suppressed.

A (poly)glycerin fatty acid ester having a number of glycerin unit(s) of 5 or less has high affinity with carotenoids such as lycopene, and a (poly)glycerin fatty acid ester having a number of fatty acid unit(s) of 6 or less has a high effect of suppressing crystals of carotenoids. Furthermore, by including a (poly)glycerin fatty acid ester containing a hydroxyl group(s) of a glycerin unit(s), a crystallization of a carotenoid can be sufficiently suppressed.

The other (poly)glycerin fatty acid ester is preferably an ester between a glycerin having a number of glycerin units (average degree of polymerization) of from 1 to 5, and more preferably from 1 to 4, and a fatty acid having a number of fatty acid units of from 1 to 6, and more preferably from 1 to 5, and having a carbon number of from 8 to 22, and more preferably a fatty acid having a carbon number of from 14 to 18, from the viewpoint of suppressing recrystallization, or the like. Examples of the fatty acid having a carbon number of from 8 to 22 include caprylic acid, capric acid, lauric acid, myristic acid, palmitic acid, stearic acid, oleic acid, linoleic acid, and behenic acid.

Among these other (poly)glycerin fatty acid esters, from the viewpoint of uniform solubility at the time of co-dissolution, a (poly)glycerin fatty acid ester having a molecular weight of 10,000 or less is preferred, a (poly)glycerin fatty acid ester having a molecular weight of 3,000 or less is more preferred, and a (poly)glycerin fatty acid ester having a molecular weight of 2,500 or less is still more preferred. Furthermore, from the viewpoint of affinity with carotenoids such as lycopene, a (poly)glycerin fatty acid ester having an HLB value of 9 or less is preferred, and a (poly)glycerin fatty acid ester having an HLB value of 6 or less is more preferred.

Examples of the other (poly)glycerin fatty acid ester that can be used in the emulsion composition of the invention include glyceryl myristate, glyceryl monostearate, diglyceryl monostearate, triglyceryl monostearate, pentaglyceryl monostearate, triglyceryl dipalmitate, glyceryl distearate, tetraglyceryl tristearate, tetraglyceryl pentastearate, hexaglyceryl tetrabehenate, and the like. From the viewpoints of suppression of recrystallization and uniform solubility, glyceryl myristate, glyceryl monostearate, diglyceryl monostearate, tetraglyceryl pentastearate, or tetraglyceryl tristearate is preferred.

The content (mass) of the other (poly)glycerin fatty acid ester is preferably from 0.01 times to 9 times, more preferably from 0.1 times to 8 times, and still more preferably from 0.3 times to 5 times, with respect to the total mass of the carotenoid from the viewpoint of stability of the emulsion composition. When the total mass of the (poly)glycerin fatty acid ester in the emulsion composition is 0.01 times the total mass of the carotenoid, a sufficient crystal suppressing effect can be expected, while when the total mass is 9 times or less, an increase in the particle diameter of the emulsion particles obtainable when an emulsion is produced, can be suppressed.

### <Other fatty acid ester>

It is preferable that the emulsion composition of the invention contains, as one of the constituent components included in the emulsion particles, at least one fatty acid ester (hereinafter, also referred to as "other fatty acid ester") selected from a triester of glycerin and a fatty acid, and an ester of an alcohol having one hydroxyl group and a fatty acid. The other fatty acid ester does not include the surfactant described above, and the (poly)glycerin fatty acid ester described above.

The preferred other fatty acid ester component is a fatty acid ester component having a total carbon number of from 10 to 60 and having no hydroxyl group in the molecule, which is at least one selected from the group consisting of a triester of glycerin and a fatty acid; and an ester of an alcohol having one hydroxyl group and a fatty acid.

Such a specific fatty acid ester component decreases the dissolution temperature of a crystalline carotenoid such as lycopene. Further, due to the fatty acid ester component, fineness of emulsified particles is more stably maintained in a case in which the emulsion composition of the invention is an oil-in-water emulsion composition.

When the total carbon number in the other fatty acid ester is 10 or more, the increase in the particle diameter of the emulsion particles tends to be further suppressed. Further, when the total carbon number is 60 or less, there is a tendency that the dissolution temperature of the crystalline carotenoid is sufficiently lowered.

From the viewpoint of lowering the dissolution temperature of a crystalline carotenoid such as lycopene, the other fatty acid ester preferably has a total carbon number of from 10 to 60, and more preferably has a total carbon number of from 27 to 57.

Further, each of the fatty acid units in the other fatty acid ester is preferably a fatty acid unit having a carbon number of from 8 to 18, more preferably a fatty acid unit having a carbon number of from 8 to 12, and still more preferably a fatty acid unit having a carbon number of from 8 to 10, from the viewpoint of suppressing a increase in the particle diameter of the emulsion particles.

The triester of glycerin and a fatty acid preferably has a total carbon number of from 10 to 60, and more preferably has a total carbon number of from 27 to 57, from the viewpoint of lowering the dissolution temperature of the crystalline carotenoid such as lycopene.

Each of the three fatty acid units in the triester of glycerin and a fatty acid is preferably a fatty acid unit having a carbon number of from 8 to 18, more preferably a fatty acid unit having a carbon number of from 8 to 12, and still more preferably a fatty acid unit having a carbon number of from 8 to 10, from the viewpoint of suppressing an increase in the particle diameter of the emulsion particles in the emulsion composition. The fatty acid unit may be a fatty acid unit derived from a saturated fatty acid, or may be a fatty acid derived from an unsaturated fatty acid. Also, the fatty acid unit may be a fatty acid unit derived from a straight-chained fatty acid, or may be a fatty acid unit derived from a branched fatty acid. Among them, from the viewpoint of lowering the dissolution temperature of a crystalline carotenoid such as lycopene, the fatty acid unit is preferably a fatty acid unit derived from a straight-chained fatty acid.

Specific examples of the triester of glycerin and a fatty acid include glyceryl tricaprylate, glyceryl tricaprate, glyceryl trilaurate, glyceryl trimyristate, glyceryl tripalmitate, glyceryl tripalmitoleate, glyceryl tristearate, glyceryl trioleate, glyceryl trilinolate, glyceryl trilinolenate, and glyceryl tri(caprylate/caprate).

From the viewpoint of lowering the dissolution temperature of the crystalline carotenoid such as lycopene, glyceryl tricaprylate, glyceryl tricaprate, glyceryl trilaurate, glyceryl tri(caprylate/caprate), and the like are preferred.

The triester of glycerin and a fatty acid may be used singly, or may be used in combination of two or more kinds thereof.

Furthermore, olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, cotton seed oil, perilla oil, soybean oil, tea seed oil, Japanese nutmeg oil, rice bran oil, Chinese tung oil, Japanese tung oil, jojoba oil, germ oil, glycerin trioctanoate, glycerin triisopalmitate, salad oil, safflower oil, coconut oil, peanut oil, almond oil, hazelnut oil, walnut oil, grape seed oil, cacao fat, palm oil, shortening, and the like, which are mixtures of trimesters of glycerin and fatty acids, can also be used.

In regard to the ester of an alcohol having one hydroxyl group and a fatty acid, an ester having a total carbon number of from 10 to 50 is preferred, and an ester having a total carbon number of from 10 to 30 is more preferred, from the viewpoint of suppressing an increase in the particle diameter of the emulsion particles.

In regard to the fatty acid unit for the ester of an alcohol having one hydroxyl group and a fatty acid, from the viewpoint of lowering the dissolution temperature of a crystalline carotenoid such as lycopene, a fatty acid unit having a carbon number of from 8 to 18 is preferred, a fatty acid unit having a carbon number of from 8 to 12 is more preferred, and a fatty acid unit having a carbon number of from 8 to 10 is still more preferred. The fatty acid unit may be a fatty acid unit derived from a saturated fatty acid, or may be a fatty acid unit derived from an unsaturated fatty acid. Furthermore, the fatty acid unit may be a fatty acid unit derived from a straight-chained fatty acid, or may be a fatty acid unit derived from a branched fatty acid. Among them, from the viewpoint of lowering the dissolution temperature of the crystalline carotenoid such as lycopene, the fatty acid unit is preferably a fatty acid unit derived from a straight-chained fatty acid.

In regard to the alcohol unit for the ester of an alcohol having one hydroxyl group and a fatty acid, an alcohol unit having a carbon number of from 2 to 35 is preferred, an alcohol unit having a carbon number of from 4 to 20 is more preferred, and an alcohol unit having a carbon number of from 5 to 15 is still more preferred, from the viewpoint of lowering the dissolution temperature of the crystalline carotenoid such as lycopene. The alcohol unit may be an alcohol unit derived from a saturated alcohol, or may be an alcohol unit derived from an unsaturated alcohol. Furthermore, the alcohol unit may be an alcohol unit derived from a straight-chained alcohol, or may be an alcohol unit derived from a branched alcohol. Among them, from the viewpoint of lowering the dissolution temperature of the crystalline carotenoid, the alcohol unit is preferably an alcohol unit derived from a straight-chained alcohol.

Examples of the ester of an alcohol having one hydroxyl group and a fatty acid include hexyl caprylate, hexyl laurate, methyl heptyl laurate, octyl dodecyl myristate, methyl heptyl isostearate, isocetyl isostearate, methyl heptyl isostearate, isopropyl isostearate, butyl stearate, 2-ethylhexyl stearate, and the like. From the viewpoint of lowering the dissolution temperature of the crystalline carotenoid such as lycopene, methyl heptyl laurate, methyl heptyl isostearate, and the like are preferred.

The ester of an alcohol having one hydroxyl group and a fatty acid may be used singly, or in combination of two or more kinds thereof.

Furthermore, regarding the fatty acid ester component, two or more kinds may be used in combination, irrespective of the kinds of the triester of glycerin and a fatty acid, and of the ester of an alcohol having one hydroxyl group and a fatty acid.

The content (mass) of the other fatty acid ester is preferably from 3 times to 300 times, more preferably from 5 times to 200 times, and still more preferably from 7 times to 100 times, the total mass of the carotenoid, from the viewpoint of lowering the dissolution temperature of the carotenoid in the emulsion composition.

When the total mass of the other fatty acid ester component in the emulsion composition of the invention is 3 times or more the total mass of the carotenoid, a sufficient effect of lowering the dissolution temperature of the carotenoid can be expected. On the other hand, when the content is 300 times or less, incorporation of a sufficient amount of the carotenoid can be achieved without impairment.

The content of the other fatty acid ester may vary with the kind or content of the other (poly)glycerin fatty acid ester used; however, from the viewpoint of stability of the emulsion composition, the content of the other fatty acid ester is preferably from 0.8 times to 750 times, more preferably from 1 time to 300 times, and still more preferably from 2 times to 100 times, with respect to the total mass of the other (poly)glycerin fatty acid ester.

When the total mass of the other fatty acid ester in the emulsion composition is 0.8 times the total mass of the other (poly)glycerin fatty acid ester, a sufficient effect of enhancing the stability of a carotenoid-containing composition can be expected. On the other hand, when the total mass of the other fatty acid ester is 750 times or less the total mass of the other (poly)glycerin fatty acid ester, incorporation of a sufficient amount of the carotenoid can be achieved without impairment.

It is preferable, from the viewpoint of suppression of crystallization of the carotenoid and stability, that the other fatty acid ester is a triester of glycerin and a fatty acid, the content (mass) of the other fatty acid ester is from 7 times to 100 times the amount of the carotenoid, the total mass of the other (poly)glycerin fatty acid ester is from 0.3 times to 5 times the total mass of the carotenoid, and the content (mass) of the other fatty acid ester is from 2 times to 100 times the total mass of the other (poly)glycerin fatty acid ester.

### <Oxidation Inhibitor>

It is preferable that the emulsion composition of the invention includes an oxidation inhibitor.

It is speculated that when the emulsion composition of the invention includes an oxidation inhibitor, decomposition of the crystalline carotenoid caused by heating (for example, oxidative decomposition) can be reliably suppressed.

As the oxidation inhibitor, for example, among the various antioxidants described in "Kosanka-zai no Riron to Jissai (Theory and Practice of Antioxidants)" (written by Kajimoto, Daisan Shobo, 1984), and the various oxidation inhibitors described in "Sanka Boshizai Handobuku (Handbook of Oxidation Inhibitors)" (written by Saruwatari, Nishino, and Tabata, Taiseisha, 1976), those functioning as oxidation inhibitors may be used. Specifically, the oxidation inhibitor is preferably at least one selected from the group consisting of a compound having a phenolic hydroxyl group, and an ascorbic acid compound. Preferable oxidation inhibitors will be exemplified below, but the invention is not intended to be limited byo these oxidation inhibitors.

Examples of the compound having a phenolic hydroxyl group include aromatic carboxylic acids, cinnamic acid compounds, ellagic acid compounds, BHT (butylhydroxytoluene), BHA(butylhydroxyanisole), and vitamin E compounds.

Examples of the aromatic carboxylic acids include gallic acid (3,4,5-hydroxybenzoic acid), and derivatives thereof. Examples of the derivatives of gallic acid (3,4,5-hydroxybenzoic acid) include gallic acid esters such as propyl gallate, epicatechin gallate, and epigallocatechin gallate; and gallic acid glycosides such as gallotannin.

Examples of the cinnamic acid compounds include ferulic acid and chlorogenic acid, and derivatives thereof. Examples of the derivatives of ferulic acid and chlorogenic acid include ferulic acid esters. Specific examples include ferulic acid, $\gamma$-orizanol (rice bran extract), caffeic acid (caffeic acid or 3,4-dihydroxycinnamic acid), chlorogenic acid, glycerylferulic acid, dihydroferulic acid, and the like.

Examples of ellagic acid compounds include ellagic acid.

Vitamin E compounds are not particularly limited, and examples thereof include those selected from a compound group including tocopherol and derivatives thereof, and a compound group including tocotrienol and derivatives thereof. These vitamin E compounds may be used singly, or in combination of plural kinds thereof. Also, a compound selected respectively from the compound group including tocopherol and derivatives thereof and the compound group including tocotrienol and derivatives thereof may be used in combination.

The compound group consisting of tocopherol and derivatives thereof include dl-a-tocopherol, dl-$\beta$-tocopherol, dl-$\gamma$-tocopherol, dl-$\delta$-tocopherol, dl-$\alpha$-tocopherol acetate, dl-$\alpha$-tocopherol nicotinate, dl-$\alpha$-tocopherol linolate, and dl-$\alpha$-

tocopherol succinate. Among these, dl-α-tocopherol, dl-β-tocopherol, dl-γ-tocopherol, di-δ-tocopherol, and mixtures thereof (mixed tocopherols) are more preferred. Furthermore, regarding the tocopherol derivatives, carboxylic acid esters, particularly acetic acid esters, of these tocopherols are preferably used.

The compound group including tocotrienol and derivatives thereof include α-tocotrienol, β-tocotrienol, γ-tocotrienol, and α-tocotrienol. Furthermore, regarding the tocotrienol derivatives, acetic acid esters of these tocotrienols are preferably used.

As the compound having a phenolic hydroxyl group, cinnamic acid compounds (for example, ferulic acid, γ-orizanol, and mixtures thereof), or vitamin E compounds (for example, a compound group including tocopherol and derivatives thereof) are preferred from the viewpoint of the stability of the carotenoid such as lycopene, and among these, at least one selected from a compound group consisting of tocopherol, tocotrienol, and derivatives thereof is more preferred.

The molecular weight of the compound having a phenolic hydroxyl group is preferably a low molecular weight from the viewpoint of the stability of the carotenoid such as lycopene. Regarding the compound having a phenolic hydroxyl group, for example, a compound having a molecular weight of from 100 to 3,000 is preferred, and a compound having a molecular weight of from 100 to 1,000 is more preferred.

The total content of the compound having a phenolic hydroxyl group in the emulsion composition of the invention may any amount effective for suppressing decomposition or loss of the carotenoid such as lycopene, and the total content can be adjusted to from 1.3 times to 15.0 times the moles of the carotenoid such as lycopene. It is preferable to adjust the total content to from 2 times to 10 times, and more preferably from 3 times to 8 times, the molar amount. When the total content of the compound having a phenolic hydroxyl group is 1.3 or more times the moles of the carotenoid such as lycopene, the amount is sufficient for manifesting the effect of suppressing a decrease in the decomposition or loss of the carotenoid. When the total content is 15.0 or less times the moles of the carotenoid, incorporation of a sufficient amount of the carotenoid can be achieved without impairment.

Examples of the ascorbic acid compounds include ascorbic acid, ascorbic acid esters, and salts thereof.

Examples of the ascorbic acid compounds include L-ascorbic acid, Na L-ascorbate, K L-ascorbate, Ca L-ascorbate, L-ascorbic acid phosphoric acid ester, magnesium salt of L-ascorbic acid phosphoric acid ester, L-ascorbic acid sulfuric acid ester, L-ascorbic acid sulfuric acid ester disodium salt, L-ascorbic acid stearic acid ester, L-ascorbic acid 2-glucoside, L-ascorbic acid palmitic acid ester, L-ascorbyl tetraisopalmitate; and fatty acid esters of ascorbic acid, such as stearic acid L-ascorbyl ester, tetraisopalmitic L-ascorbyl ester, and palmitic acid L-ascorbyl ester. Among these ascorbic acid compounds, L-ascorbic acid, Na L-ascorbic acid, Ca L-ascorbic acid, L-ascorbic acid stearic acid ester, L-ascorbic acid 2-glucoside, L-ascorbic acid palmitic acid ester, magnesium salt of L-ascorbic acid phosphoric acid ester, L-ascorbic acid sulfuric acid ester disodium salt, and L-ascorbyl tetraisopalmitate are particularly preferred from the viewpoint of suppressing thermal loss of the carotenoid.

These ascorbic acid compounds may be included in the oil phase composition in the form of simple substance, or may be incorporated in the oil phase composition in the form of an aqueous solution. The concentration of the ascorbic acid compound in such an aqueous solution is not particularly limited, but it is generally preferable to set the concentration to from 0.05% by mass to 5% by mass, from the viewpoint of preventing oxidation.

The total content of the ascorbic acid compound in the emulsion composition of the invention is preferably from 0.05 times to 50 times, more preferably from 1 time to 10 times, still more preferably from 1.5 times to 10 times, and still more preferably from 2 times to 10 times, the mass of the carotenoid such as lycopene, from the viewpoint of suppressing heat-induced loss of the carotenoid. When the mass of the ascorbic acid compound is 0.05 or more times the amount of the crystalline carotenoid including lycopene, it is sufficient for manifesting an effect of suppressing a decrease in the crystalline carotenoid content. When the mass of the ascorbic acid compound is 50 times or less, incorporation of a sufficient amount of the crystalline carotenoid can be achieved without impairment.

In the emulsion composition of the invention, the oxidation inhibitor may be used singly, or may be used in combination of two or more kinds thereof.

Furthermore, when a compound having a phenolic hydroxyl group and an ascorbic acid compound are both used as oxidation inhibitors, it is preferable because decomposition of the carotenoid such as lycopene caused by heating (for example, oxidative decomposition) can be reliably suppressed, and a reduction of the amount of the carotenoid during the production process for the emulsion composition can be suppressed.

**<Other oily components>**

As the component that constitutes the emulsion particles in the emulsion composition of the invention, the emulsion composition may further include other oily components besides the various components previously mentioned above.

Regarding the other oily components, any component which has a solubility in water at 25°C of less than 0.5% by mass, and dissolves in the oil phase components including the carotenoid according to the invention at 90°C in an amount of 5% by mass or more, may be used without any particular limitations, and any such compound having properties or functionality according to the purpose can be appropriately selected and used. For example, non-crystalline carotenoids, ubiquinones, oil-soluble vitamins, squalane, and squalene are preferably used.

Examples of the ubiquinones include co-enzyme Q compounds such as co-enzyme Q10.

Furthermore, examples of the oil-soluble vitamins include fatty acid esters of erythorbic acid, such as palmitic acid erythorbyl ester and tetraisopalmitic acid erythorbyl ester; and fatty acid esters of vitamin B6, such as pyridoxine dipalmitate, pyridoxine tripalmitate, pyridoxine dilaurate, and pyridoxine dioctanoate.

### <Preferred embodiments of oil-in-water emulsion composition>

The emulsion composition of the invention contains emulsion particles having an average particle diameter of 120 nm or less. When the particle diameter of the emulsion particles is 120 nm or less, the emulsion composition of the invention has excellent microstability of the emulsion particles and excellent transparency.

The average particle diameter of the emulsion particles according to the invention is 120 nm or less from the viewpoint of transparency and from the viewpoint of absorbability into the living body, and is more preferably 100 nm or less, and preferably from 10 nm to 100 nm. From the viewpoint of transparency, the average particle diameter is more preferably from 25 nm to 90 nm, and most preferably from 40 nm to 80 nm.

In view of the particle diameter range and ease of measurement, the method of measuring the average particle diameter of the emulsion particles in the invention is a dynamic light scattering method. Examples of commercially available analyzers utilizing dynamic light scattering include a NANOTRAC UPA (Nikkiso Co., Ltd.), a dynamic light scattering type particle size distribution analyzer, LB-550 (Horiba, Ltd.), and a concentrated system particle size analyzer, FPAR-1000 (Otsuka Electronics Co, Ltd.); however, for the particle size according to the invention, a value measured at 25°C using a particle size analyzer, FPAR-1000 (Otsuka Electronics Co., Ltd.) is employed. Specifically, the emulsion composition is diluted with pure water as necessary so as to obtain an oil phase concentration of 0.5% by mass, and the particle size is determined as the median diameter (d = 50) using a particle size analyzer "FPAR-1000 (Otsuka Electronics Co., Ltd.)".

Furthermore, the average particle diameter of the emulsion particles can be adjusted by factors such as the stirring conditions for the production method (shear force, temperature, and pressure), and the ratio of the oil phase and the aqueous phase besides the components in the composition.

The emulsion composition of the invention is an oil-in-water emulsion composition obtainable by emulsifying and mixing an aqueous phase composition composed of aqueous phase components and an oil phase composition composed of oil phase components.

The content of the oil phase composition in the oil-in-water emulsion composition is preferably from 0.1% by mass to 50% by mass, more preferably from 0.5% by mass to 30% by mass, and still more preferably from 3% by mass to 25% by mass, from the viewpoint of exhibiting the functions of the oily components.

The oil phase composition is preferably a composition which includes a carotenoid (more preferably, lycopene) and a higher fatty acid, and further includes other oily components selected as desired. Furthermore, the aqueous composition is preferably an aqueous composition which is configured to include an aqueous medium, particularly water, and includes at least a surfactant that functions as an emulsifier.

### <Other additive components>

In addition to the various components described above, components that are conventionally used in the fields of foods, cosmetics and the like may be appropriately incorporated into the emulsion composition of the invention according to the form of the relevant composition. The additive components may be incorporated as the components of the oil phase composition or the components of the aqueous phase composition, depending on the characteristics of the additive components, or may be incorporated as additive components to the aqueous phase after the production of the emulsion composition.

Examples of such other components include polyhydric alcohols such as glycerin and 1,3-butylene glycol; monosaccharides or polysaccharides such as glucose, fructose, lactose, maltose, sucrose, pectin, kappa-carrageenan, locust bean gum, guar gum, hydroxypropyl guar gum, xanthan gum, karaya gum, tamarind seed polysaccharides, gum arabic, tragacanth gum, hyaluronic acid, sodium hyaluronate, sodium chondroitin sulfate, and dextrin; sugar alcohols such as sorbitol, mannitol, maltitol, lactose, maltotriitol, and xylytol; inorganic salts such as sodium chloride and sodium sulfate; proteins having molecular weights of more than 5000, such as casein, albumin, methylated collagen, hydrolyzed collagen, water-soluble collagen, and gelatin; synthetic polymers such as carboxyvinyl polymers, polysodium acrylate, polyvinyl alcohol, polyethylene glycol, and ethylene oxide-propylene oxide block copolymers; water-soluble cellulose derivatives such as hydroxyethyl cellulose and methyl cellulose; flavonoids (catechin, anthocyanin, flavones, isoflavone, flavane, flavanone, and rutin), lignans, curcumins, and coumarins. These additive components may be include in the emulsion composition as, for example, functional components, excipients, viscosity adjusting agents, and radical scavengers, based on their functions.

In addition to them, for example, various efficacious components, a pH adjusting agent, a pH buffering agent, an ultraviolet absorber, a fragrance, and a colorant, that is, other additives that are conventionally used for those applications can be used in combination.

The emulsion composition of the invention may include an antiseptic agent and a preservative; however, from the viewpoint of stably maintaining the emulsified state, it is preferable that the content of the antiseptic agent and preservative in the composition is 0.001% by mass or less.

Here, the antiseptic agent and preservative refer to various components that are incorporated for the purpose of suppressing growth of microorganisms in the composition. Specifically, the antiseptic agent and preservative refer to at least one selected from the group defined by Japan Ministry of Health and Welfare Notification 331 "Standards for Cosmetics" and Japan Ministry of Health and Welfare Notification No. 370 "Standards and criteria for food and food additives".

The emulsion composition of the invention is an emulsion composition from which a desired effect induced by carotenoids is sufficiently expected since decomposition of carotenoids is suppressed, and storage stability is excellent. Therefore, the emulsion composition of the invention can be preferably applied to food compositions, cosmetic compositions, pharmaceutical compositions, and the like.

Furthermore, components that can be added to foods or cosmetics can be appropriately added, if necessary, to foods or cosmetics that contain the emulsion composition of the invention.

Foods, cosmetics and the like that contain the emulsion composition of the invention may exhibit an effect that may not be sufficiently exhibited due to decomposition of carotenoids or deterioration of an emulsion state, for example, satisfactory absorbability of carotenoids.

The emulsion composition of the invention is suitably used in, for example, skin toners, essences, emulsions, cream packs/masks, packs, cosmetics for hair cleaning, fragrance cosmetics, liquid body cleanser, UV care cosmetics, deodorant cosmetics, oral care cosmetics, and the like.

Also, the emulsion composition of the invention is also suitably used in, as foods, general food products such as nutritional drinks, revitalizers, palatable beverages, and frozen desserts; as well as in nutrition supplement foods in capsule forms, and the like.

The emulsion composition of the invention can be produced by known methods. Regarding a preferred production method of the emulsion composition of the invention, the production method described below may be used.

**[Method of producing emulsion composition]**

The method of producing the emulsion composition of the invention includes mixing an oil phase composition containing a carotenoid such as lycopene with an aqueous phase composition, and emulsifying the mixture under pressure.

According to a preferred embodiment for obtaining an oil phase composition containing a carotenoid such as lycopene, an embodiment is available that includes heating, under temperature conditions of 90°C or higher, an oil phase component mixed liquid including a carotenoid such as lycopene; a higher fatty acid having a total carbon number of 12 or more; a (poly)glycerin fatty acid ester having a number of glycerin units of from 1 to 5, a number of fatty acid units of from 1 to 6, and at least one hydroxyl group of a glycerin unit; another fatty acid ester component; and an oxidation inhibitor.

Hereinafter, the method of preparing an emulsion composition will be described in detail with reference to an example of the method of preparing an emulsion composition using an oil phase composition of the present embodiment, but the invention is not intended to be limited to this.

According to the production method of the present embodiment, since a carotenoid such as lycopene is heated together with a predetermined (poly)glycerin fatty acid ester, a predetermined fatty acid ester component, and an oxidation inhibitor under conditions at a temperature higher than or equal to the dissolution temperature of the carotenoid, the carotenoid component co-dissolves with the predetermined (poly)glycerin fatty acid ester and the predetermined fatty acid ester component. When this lycopene-containing oil phase composition obtained by co-dissolution is used as the oil phase composition that is to be heated and emulsified together with an aqueous phase composition containing a water-soluble emulsifier, the emulsion composition obtained by emulsification becomes a composition in which crystallization of lycopene, which is a crystalline carotenoid, is suppressed. The production method of the present embodiment constitutes addition of the predetermined fatty acid ester components. Thereby, it is possible to lower the dissolution temperature of a carotenoid such as lycopene, as compared with a case in which the predetermined fatty acid ester components are not added.

According to the production method of the present embodiment, first, a carotenoid; a (poly)glycerin fatty acid ester having a number of glycerin units of from 1 to 5, a number of fatty acid units of from 1 to 6, and at least one hydroxyl group of a glycerin unit; another fatty acid ester component; and an oxidation inhibitor are mixed, whereby an oil phase component mixed liquid is obtained (hereinafter, also referred to as "oil phase component mixing process"). The oil phase component mixed liquid may include other oil phase components as necessary.

Specific examples of the carotenoid, (poly)glycerin fatty acid ester, other fatty acid ester component, and oxidation inhibitor that are included in the oil phase component mixed liquid used in the oil phase component mixing process, for example, the contents and preferred ranges thereof, are the same as described in connection with the various components included in the emulsion composition of the invention.

Thereafter, the oil phase component mixed liquid is heated under the temperature conditions at 90°C or higher, an oil phase composition is obtained (hereinafter, also referred to as "oil phase component heating process"). The heating temperature may be any temperature higher than or equal to 90°C, and can be set to 90°C to 155°C. From the viewpoint of suppressing thermal decomposition of the carotenoid, the heating temperature is preferably from 110°C to 150°C, and more preferably from 120°C to 145°C.

The heating time for the oil phase component heating process is desirably a time period in which the carotenoid in the oil phase component mixed liquid is dissolved, and from the viewpoint of efficiently suppressing the non-crystallization of a crystalline body and excessive decomposition of the carotenoid caused by heat, the heating time is preferably from 1 minute to 60 minutes, and more preferably from 5 minutes to 45 minutes; however, the heating time is not limited to this.

An oil phase composition is obtained from an oil phase component mixed liquid containing a carotenoid by such a heating treatment.

In addition, regarding the heating treatment, since it is preferable to make the entire oil phase component mixed liquid to be at a uniform temperature, it is preferable to sufficiently stir the oil phase component mixed liquid while heating, and it is desirable to heat the mixed liquid with stirring in a sealed container, and to maintain the mixed liquid at a constant temperature.

An oil phase composition is obtained by the oil phase component heating process.

After the oil phase component heating process, emulsifying the oil phase composition obtained by the oil phase component heating process with an aqueous phase composition (emulsifying process) is included. Thereby, an oil-in-water emulsion composition in which the oil phase components including a carotenoid are finely dispersed as oil droplets (emulsion particles) in water is obtained. In this composition, the carotenoid is stably maintained.

The ratio (mass) between the oil phase and the aqueous phase for emulsification is not particularly limited, but the oil phase/aqueous phase ratio (% by mass) is preferably from 0.1/99.9 to 50/50, more preferably from 0.5/99.5 to 30/70, and still more preferably from 1/99 to 20/80.

When the oil phase/aqueous phase ratio is adjusted to 0.1/99.9 or higher, since the level of the active ingredients is not too low, there is a tendency that no problem occurs in the practical use of the oil-in-water emulsion composition, which is preferable. Furthermore, when the oil phase/aqueous phase ratio is adjusted to 50/50 or lower, the emulsifier concentration is not diluted, and there is a tendency that the emulsification stability of the emulsion composition is not deteriorated, which is preferable.

In regard to pressurized emulsification, it is acceptable to carry out a single-process emulsification operation; however, it is preferable to carry out an emulsification operation of two or more processes, from the viewpoint of obtaining uniform and fine emulsion particles.

Specifically, it is particularly preferable to use two or more kinds of emulsifying apparatuses in combination by a method of performing emulsification through a high pressure homogenizer or the like, in addition to a single-process emulsification operation of performing emulsification using a conventional emulsifying apparatus that utilizes a shearing action (for example, a stirrer, an impeller stirrer, a homomixer, or a continuous flow type shearing apparatus). When a high pressure homogenizer is used, the emulsification product can be arranged into liquid droplets of more uniform fine particles. Also, the operation may be carried out several times for the purpose of making liquid droplets having an still more uniform particle diameter.

Regarding the emulsification means that can be used herein, generally known emulsification methods such as a natural emulsification method, an interface chemistry-applicable emulsification method, an electric emulsification method, a capillary emulsification method, a mechanical emulsification method, and an ultrasonic emulsification method, can all be used.

As useful methods for further micronizing the emulsion particles in the emulsion composition, interface chemistry-applicable methods such as a PIT emulsification method and a gel emulsification method are known. These methods are advantageous in that less energy is consumed, and are adequate in the case of finely emulsifying a material that is susceptible to thermal deterioration.

Furthermore, as an emulsification method that is commonly used, a method of using mechanical force, that is, a method of breaking oil droplets by applying a strong shear force from an external source is applied. The most common example of the mechanical force is high speed, high shear stirring machine. Commercially available examples of such a stirring machine include stirring machines called a Homomixer, a Disper mixer, and an Ultramixer.

Furthermore, available as another mechanical emulsifying apparatus that is useful for micronization is a high pressure homogenizer, and various apparatuses are commercially available. Since a high pressure homogenizer can provide a large shear force compared with a stirring system, micronization can be achieved even if the amount of the emulsifier is relatively small.

High pressure homogenizers can be roughly divided into chamber type high pressure homogenizers having a fixed throttle unit, and homogenizing valve type high pressure homogenizers for which the degree of opening of the throttle is controlled.

Examples of the chamber type high pressure homogenizers include a MICROFLUIDIZER (manufactured by Microfluidics Corp.), a NANOMIZER (manufactured by Yoshida Kikai Co., Ltd.), and an ULTIMIZER (manufactured by Sugino Machine, Ltd.).

Examples of the homogenizing valve type high pressure homogenizers include a Gaulin type Homogenizer (manufactured by APV Corp.), a Lanier type homogenizer (manufactured by Lanier Co., Ltd.), a high pressure homogenizer (manufactured by GEANiro Soavi S.p.A.), a homogenizer (manufactured by Sanwa Machinery Trading Co., Ltd.), a high pressure homogenizer (manufactured by Izumi Food Machinery Co., Ltd.), and an ultrahigh pressure homogenizer (manufactured by IKA Group).

Regarding a dispersing apparatus having relatively high energy efficiency, there is available an ultrasonic homogenizer as an emulsifying apparatus having a simple structure. Examples of the high power output ultrasonic homogenizer capable of production include ULTRASONIC HOMOGENIZERS US-600, US-1200T, RUS-1200T, and MUS-1200T (all manufactured by Nissei Corp.); and ULTRASONIC PROCESSORS UIP2000, UIP-4000, UIP-8000, and UIP-16000 (all manufactured by Hielscher Ultrasonics GmbH). These high power output ultrasonic irradiation apparatuses are used at a frequency of 25 kHz or less, and preferably from 15 kHz to 20 kHz.

Regarding another known emulsifying means, methods of using a static mixer, a microchannel, a micromixer, or a membrane emulsifying apparatus, which do not have a stirring unit from the outside and require only a low level of energy, are also useful.

There are no particular limitations on the temperature conditions at the time of performing emulsifying dispersion according to the production method of the present embodiment; however, from the viewpoint of stability of the functional oily components, the temperature is preferably from 10°C to 100°C, and an appropriately preferred range can be selected depending on the melting point of the handled functional oily component, or the like.

Furthermore, in the case of using a high pressure homogenizer in the invention, it is preferable to handle the process at a pressure of preferably 50 MPa or higher, more preferably from 50 MPa to 280 MPa, and still more preferably from 100 MPa to 280 MPa.

The emulsion liquid which is an emulsifying dispersed composition is preferably cooled through a certain cooler within 30 seconds, and preferably within 3 seconds, immediately after the passage through the chamber, from the viewpoint of retaining the particle size of the dispersed particles.

The emulsion composition obtained as described above is preferably subjected to a heating treatment by an arbitrary heating means, from the viewpoint of enhancing storability. The heating temperature for the heating treatment is preferably 70°C or higher, and more preferably from 75°C to 85°C. Also, the heating time can be appropriately set depending on the composition, amount, and the like of the emulsion composition to be treated, and can be adjusted to about from 10 minutes to 90 minutes.

One suitable embodiment according to the invention is an embodiment including heating the emulsion composition of the invention to 70°C or higher after preparation of the emulsion composition, immediately before or after filling a storage container with the emulsion composition; and sealing a storage container that has been filled with the emulsion composition that has been heated. When the emulsion composition is heated to 70°C or higher immediately before or after filling a storage container, the storability of the emulsion composition can be enhanced.

Filling of the emulsion composition into a storage container, which is performed before or after heating of the emulsion composition, may be carried out using a known filling method.

Regarding the storage container, there are no particular limitations as long as the storage container has heat resistance with respect to heating treatment, and has sufficient strength to retaining the emulsion composition in a sealed state, and any sealable container having a given shape and formed from a known material such as glass or a resin can be used.

The emulsion composition obtained as described above may be used in various applications at the concentration obtained after production, or may be used in various applications after the emulsion composition is diluted to a predetermined concentration by adding thereto a water-based medium such as water and arbitrary additives that are used as desired.

Dilution of the emulsion composition may be carried out by directly adding a water-based medium such as water and arbitrary additives, that are used as desired, to the emulsion composition separately, or may be carried out by separately preparing an aqueous solution for dilution containing a water-based medium such as water and arbitrary additives that are used as desired, and adding the aqueous solution for dilution to the emulsion composition.

On the occasion of diluting the emulsion composition, it is preferable to add an ascorbic acid compound to the emulsion composition from the viewpoint of maintaining the storage stability of the emulsion composition after dilution. Regarding the ascorbic acid compound, the compounds described above as oxidation inhibitors can be used.

## EXAMPLES

Hereinafter, the invention will be explained by way of Examples, but the invention is not intended to be limited to these. Note that, the "parts" and "%" in the following description are based on mass unless specified otherwise.

**[Example 1]**

A lycopene-containing emulsion composition of Example 1 was prepared as follows.

An oil phase composition described below was heated and mixed for 5 minutes while stirred on a hot plate at 135°C, and it was confirmed that the composition was thoroughly mixed.

An aqueous phase composition described below was heated and mixed while stirred in a constant temperature bath at 70°C, and it was confirmed that the composition was thoroughly mixed. The aqueous phase composition was maintained at 70°C.

The aqueous phase composition was added to the oil phase composition and mixed with stirring, and then the mixture was dispersed using an ultrasonic homogenizer. Thereafter, a crude dispersion product thus obtained was further subjected to high pressure emulsification at 200 MPa using an ultrahigh pressure emulsifying apparatus (ULTIMIZER, manufactured by Sugino Machine, Ltd.)

**- Oil phase composition -**

| (1) Lycopene[*1] | 0.17 parts |
|---|---|
| (2) Glycerin tri(caprylate/caprate)[*2] | 13.9 parts |
| (3) Mixed tocopherol[*3] | 0.64 parts |
| (4) Diglyceryl monostearate[*4] | 0.28 parts |
| (5) Isostearic acid[*5] | 0.00017 parts |

**- Aqueous phase composition -**

| (1) Decaglyceryl oleate[*6] | 10.0 parts |
|---|---|
| (2) Glycerin | 45.0 parts |
| (3) Purified water | 30.0 parts |
| *1: manufactured by Wako Pure Chemical Industries, Ltd. | |
| *2: "COCONARD MT" (HLB = 1) manufactured by Kao Corp. | |
| *3: "RIKEN E OIL 800" manufactured by Riken Vitamin Co., Ltd. | |
| *4: "NIKKOL DGMS" (HLB = 5.0) manufactured by Nikko Chemicals Co., Ltd. | |
| *5: "ISOSTEARIC ACID EX" manufactured by Kokyu Alcohol Kogyo Co., Ltd. | |
| *6: DECAGLYN 1-O manufactured by Nikko Chemicals Co., Ltd. | |

**[Examples 2 to 9, 11 and 12, Reference Examples 10, 13 and 14 and Comparative Examples 1 to 5]**

In regard to Examples 2 to 8, 11 and 12 and Reference Examples 10, 13 and 14, and Comparative Examples 1 to 5, lycopene-containing emulsion compositions were obtained in the same manner as in Example 1, except that the kinds and contents of the various components used in the oil phase composition and the aqueous phase composition in Example 1 were changed as shown in Table 1.

In regard to Example 9, a lycopene-containing emulsion composition was obtained in the same manner as in Example 1, except that the kinds and contents of the various components used in the oil phase composition and the aqueous phase composition in Example 1 were changed as shown in Table 1, and the pressure for high pressure emulsification was changed to 100 MPa.

The values representing the amounts of incorporation of the various components in Table 1 represent "parts". The following compounds were used for the various components described in Table 1.

- Oleic acid: "LUNAC O-V" manufactured by Kao Corp.
- Lauric acid: manufactured by Wako Pure Chemical Industries, Ltd.
- Palmitic acid: manufactured by Wako Pure Chemical Industries, Ltd.
- Behenic acid: manufactured by Wako Pure Chemical Industries, Ltd.
- Sucrose oleic acid ester: "RYOTO SUGAR ESTER O-1570" manufactured by Mitsubishi Kagaku Foods Corp.
- Glycerin: "Purified Glycerin" manufactured by Kao Corp.

**<Evaluation>**

For the respective lycopene-containing emulsion compositions of the Examples, Reference Examples and Comparative Examples obtained as described above, (1) measurement of the emulsion particle diameter immediately after production, (2) measurement of the emulsion particle diameter after heating treatment, and (3) evaluation of the lycopene decomposition ratio, were carried out.

The evaluation results are described together in Table 1. In Table 1, "-" indicates that a component was not incorporated.

**(1) Measurement of emulsion particle diameter immediately after production**

Purified water was added to each of the emulsion compositions of the Examples and Comparative Examples immediately after production obtained as described above, and a 1% dilution liquid was prepared. The average particle diameter (median particle diameter) on a volume basis of the emulsion particles in the dilution liquid was measured with a dynamic light scattering meter (trade name: FPAR-1000, manufactured by Otsuka Electronics Co., Ltd.)

**(2) Measurement of emulsion particle diameter after heating treatment**

Each of the emulsion compositions of the Examples and Comparative Examples obtained as described above was subjected to a heating treatment at 80°C for 60 minutes. Purified water was added to the emulsion composition after the heating treatment, and a 1% dilution liquid was prepared. The average particle diameter (median particle diameter) on a volume basis of the emulsion particles in the dilution liquid was measured with a dynamic light scattering meter (trade name: FPAR-1000, manufactured by Otsuka Electronics Co., Ltd.).

**(3) Lycopene decomposition ratio**

Each of the emulsion compositions of the Examples and Comparative Examples obtained as described above was subjected to a heating treatment at 80°C for 60 minutes. 0.3 parts of the emulsion composition after heating treatment, 1 part of sodium citrate, 1 part of magnesium ascorbyl phosphate, and 99.7 parts of a 10 mM phosphate buffer aqueous solution (pH 7) were mixed and stirred, and thus the emulsion composition was diluted. Each of the emulsion compositions after dilution was filled into a 5-ml glass vial, and the vial was sealed and stored for 2 weeks at 50°C.

For each of the emulsion compositions immediately after production and after storage for 2 weeks, the absorbance was measured according to the following measurement conditions, and the lycopene decomposition ratio (%) was calculated by the following formula:

$$\text{Decomposition ratio (\%)} = (\text{Absorbance immediately after production - absorbance after storage for 2 weeks at 50°C}) / \text{absorbance immediately after production} \times 100$$

A smaller decomposition ratio (%) represents superior stability over time.

**(Measurement conditions for absorbance)**

The emulsion compositions were diluted with pure water such that the lycopene concentration in the emulsion composition would be 0.0004% by mass. For each of the emulsion compositions after dilution, the absorbance at 510 nm was measured in a 10-mm cell using a UV-VISIBLE Spectrophotometer, UV-2550 (manufactured by Shimadzu Corp.).

[Table 1]

(Parts)

| | | Comparative Example 1 | Comparative Example 2 | Comparative Example 3 | Comparative Example 4 | Comparative Example 5 | Example 1 | Example 2 | Example 3 | Example 4 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|---|---|---|---|---|
| Oil phase composition | Lycopene | 0.17 | 0.17 | 0.17 | 0.17 | 0.17 | 0.17 | 0.17 | 0.17 | 0.17 | 0.17 | 0.17 | 0.17 | 0.17 | 0.17 | 0.17 | 0.17 | 0.17 | 0.17 | 0.17 |
| | COCONARD MT | 13.9 | 13.9 | 13.1 | 13.9 | 13.9 | 13.9 | 13.9 | 13.8 | 13.6 | 13.9 | 13.9 | 13.9 | 13.9 | 13.9 | 13.9 | 13.9 | 13.9 | 13.9 | 13.9 |
| | Mixed tocopherol | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 | 0.64 |
| | Diglycerin monostearate | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 | 0.28 |
| | Isostearic acid | - | 0.000034 | 0.85000 | 0.0017 | 0.00170 | 0.00017 | 0.00170 | 0.08500 | 0.34000 | - | - | - | - | 0.00170 | 0.00170 | 0.00170 | 0.00170 | 0.00170 | 0.00170 |
| | Oleic acid | - | - | - | - | - | - | - | - | - | 0.00170 | - | - | - | - | - | - | - | - | - |
| | Lauric acid | - | - | - | - | - | - | - | - | - | - | 0.00170 | - | - | - | - | - | - | - | - |
| | Palmitic acid | - | - | - | - | - | - | - | - | - | - | - | 0.00170 | - | - | - | - | - | - | - |
| | Behenic acid | - | - | - | - | - | - | - | - | - | - | - | - | 0.00170 | - | - | - | - | - | - |
| Aqueous phase composition | Decaglyceryl oleate | 10.0 | 10.0 | 10.0 | 4.0 | 20.0 | 10.0 | 10.0 | 10.0 | 10.0 | 10.0 | 10.0 | 10.0 | 10.0 | 10.0 | 4.0 | 6.0 | 15.0 | 20.0 | 6.0 |
| | Sucrose oleic acid ester | - | - | - | - | - | - | - | - | - | - | - | - | - | - | - | - | - | - | 6.0 |
| | Glycerin | 45.0 | 45.0 | 45.0 | 48.6 | 39.0 | 45.0 | 45.0 | 45.0 | 45.0 | 45.0 | 45.0 | 45.0 | 45.0 | 45.0 | 48.6 | 47.4 | 39.0 | 39.0 | 47.4 |
| | Water | 30.0 | 30.0 | 30.0 | 52.4 | 26.0 | 30.0 | 30.0 | 30.0 | 30.0 | 30.0 | 30.0 | 30.0 | 30.0 | 30.0 | 32.4 | 31.6 | 26.0 | 26.0 | 31.6 |
| Amount of higher fatty acid / amount of lycopene | | - | 0.0002 | 5.000 | 0.010 | 0.010 | 0.001 | 0.010 | 0.500 | 2.000 | 0.010 | 0.010 | 0.010 | 0.010 | 0.010 | 0.010 | 0.010 | 0.010 | 0.010 | 0.010 |
| Particle diameter immediately after production | | 65 nm | 65 nm | 67 nm | 30 nm | 135 nm | 63 nm | 61 nm | 61 nm | 61 nm | 62 nm | 65 nm | 64 nm | 61 nm | 110 nm | 115 nm | 81 nm | 70 nm | 108 nm | 109 nm |
| Particle diameter after heating treatment at 80°C for 60 minutes | | 140 nm | 120 nm | 67 nm | 33 nm | 134 nm | 75 nm | 63 nm | 62 nm | 62 nm | 62 nm | 72 nm | 69 nm | 62 nm | 111 nm | 117 nm | 82 nm | 70 nm | 110 nm | 114 nm |
| Lycopene decomposition ratio after storage for 2 weeks at 50°C after dilution | | 26% | 22% | 24% | 22% | 23% | 12% | 9% | 10% | 12% | 9% | 13% | 13% | 9% | 3% | 14% | 10% | 11% | 13% | 14% |

In Table 1, Examples 10, 13 and 14 are Reference Examples.

As shown in Table 1, it is understood that all of the respective lycopene-containing emulsion compositions of Examples 1 to 9, 11 and 12 and Reference Examples 10, 13 and 14 can stably maintain an emulsified state with respect to heating treatment, and have excellent storage stability.

Furthermore, it is understood from Examples 2, 11 and 12 and Reference Examples 10 and 13 that the content of the surfactant other than the higher fatty acid is more preferably from 6% by mass to 15% by mass. Furthermore, it is understood from a comparison of Example 11 and Reference Example 14, that a polyglycerin fatty acid ester is more preferred as the surfactant other than the higher fatty acid.

## Claims

1. A carotenoid-containing oil-in-water emulsion composition, comprising:

a carotenoid;
a higher fatty acid having a total carbon number of 12 or more in an amount of from 0.001 times to 2 times by mass ratio with respect to the carotenoid; and
a surfactant other than the higher fatty acid having a total carbon number of 12 or more, wherein an average particle diameter of emulsion particles, which is the D50, measured using the dynamic light scattering method described in the description, is 120 nm or less, wherein a content of the surfactant other than the higher fatty acid having a total carbon number of 12 or more is from 6% by mass to 15% by mass with respect to the total mass of the emulsion composition, and
wherein the surfactant is a polyglycerin fatty acid ester having an HLB value of from 10 to 20 and having 6 or more glycerin units.

2. The carotenoid-containing oil-in-water emulsion composition according to claim 1, wherein the carotenoid is lycopene.

3. The carotenoid-containing oil-in-water emulsion composition according to claim 1 or 2, comprising the higher fatty acid having a total carbon number of 12 or more in an amount of from 0.01 times to 0.5 times by mass ratio with respect to the carotenoid.

4. The carotenoid-containing oil-in-water emulsion composition according to any one of claims 1 to 3, wherein the average particle diameter of the emulsion particles is 100 nm or less.

5. The carotenoid-containing oil-in-water emulsion composition according to any one of claims 1 to 4, wherein the higher fatty acid having a total carbon number of 12 or more is a higher fatty acid having a total carbon number of from 18 to 22.

6. The carotenoid-containing oil-in-water emulsion composition according to any one of claims 1 to 5, wherein a total content of an antiseptic agent and a preservative is 0.001% by mass or less with respect to the total mass of the emulsion composition.

7. A method of producing an emulsion composition, the method comprising:

preparing the carotenoid-containing oil-in-water emulsion composition according to any one of claims 1 to 6;
subsequently heating the emulsion composition to 70°C or higher immediately before or after filling a storage container with the emulsion composition; and sealing the storage container that has been filled with the heated emulsion composition.

## Patentansprüche

1. Carotinoid enthaltende Öl-in-Wasser Emulsionszusammensetzung, enthaltend:

ein Carotinoid;
eine höhere Fettsäure mit einer Gesamtkohlenstoffanzahl von 12 oder mehr in einer Menge von einem 0,001-Fachen bis 2-Fachen des Masseverhältnisses bezogen auf das Carotinoid; und
ein Tensid, das von der höheren Fettsäure mit einer Gesamtkohlenstoffanzahl von 12 oder mehr unterschiedlich ist, wobei ein durchschnittlicher Partikeldurchmesser der Emulsionspartikel, der dem D50 entspricht, gemessen unter Verwendung des in der Beschreibung beschriebenen dynamischen Lichtstreuverfahrens, 120 nm oder weniger beträgt, wobei ein Gehalt an dem Tensid, das von der höheren Fettsäure mit einer Gesamtkohlenstoffanzahl von 12 oder mehr unterschiedlich ist, von 6 Masse% bis 15 Masse%, bezogen auf die Gesamtmasse der Emulsionszusammensetzung, beträgt, und
wobei das Tensid ein Polyglycerinfettsäureester mit einem HLB-Wert von 10 bis 20 ist und 6 oder mehr Glycerineinheiten umfasst.

2. Carotinoid enthaltende Öl-in-Wasser Emulsionszusammensetzung nach Anspruch 1, wobei das Carotinoid Lycopen ist.

3. Carotinoid enthaltende Öl-in-Wasser Emulsionszusammensetzung nach Anspruch 1 oder 2, umfassend die höhere Fettsäure mit einer Gesamtkohlenstoffanzahl von 12 oder mehr in einer Menge von einem 0,01-Fachen bis 0,5-Fachen des Masseverhältnisses bezogen auf das Carotinoid.

4. Carotinoid enthaltende Öl-in-Wasser Emulsionszusammensetzung nach einem der Ansprüche 1 bis 3, wobei der durchschnittliche Partikeldurchmesser der Emulsionspartikel 100 nm oder weniger beträgt.

5. Carotinoid enthaltende Öl-in-Wasser Emulsionszusammensetzung nach einem der Ansprüche 1 bis 4, wobei die höhere Fettsäure mit einer Gesamtkohlenstoffanzahl von 12 oder mehr eine höhere Fettsäure mit einer Gesamtkohlenstoffanzahl von 18 bis 22 ist.

6. Carotinoid enthaltende Öl-in-Wasser Emulsionszusammensetzung nach einem der Ansprüche 1 bis 5, wobei ein Gesamtgehalt an antiseptischem Mittel und Konservierungsmittel 0,001 Masse% oder weniger bezogen auf die Gesamtmasse der Emulsionszusammensetzung beträgt.

7. Verfahren zur Herstellung einer Emulsionszusammensetzung, wobei das Verfahren umfasst:

   Herstellen der Carotinoid enthaltenden Öl-in-Wasser Emulsionszusammensetzung nach einem der Ansprüche 1 bis 6;
   anschließendes Erwärmen der Emulsionszusammensetzung auf 70°C oder mehr unmittelbar vor oder nach dem dem Befüllen eines Aufbewahrungsbehältnisses mit der Emulsionszusammensetzung; und Versiegeln des Aufbewahrungsbehältnisses, das mit der erwärmten Emulsionszusammensetzung befüllt wurde.

**Revendications**

1. Composition d'émulsion huile dans eau contenant un caroténoïde, comprenant :

   un caroténoïde ;
   un acide gras supérieur présentant un nombre total de carbone supérieur ou égal à 12 en une quantité allant de 0,001 fois à 2 fois quant au rapport de masse, par rapport au caroténoïde, et
   un tensio-actif autre que l'acide gras supérieur présentant un nombre total de carbone supérieur ou égal à 12, dans laquelle un diamètre de particules moyen de particules d'émulsion, lequel est le D50, mesuré à l'aide du procédé de diffusion dynamique de la lumière décrit dans la description, est inférieur ou égal à 120 nm, dans laquelle une teneur du tensio-actif autre que l'acide gras supérieur présentant un nombre total de carbone supérieur ou égal à 12 s'étend de 6 % en masse à 15 % en masse par rapport à la masse totale de la composition d'émulsion, et
   dans laquelle le tensio-actif est un ester polyglycérolique d'acides gras présentant une valeur HLB (balance hydrophile-lipophile) allant de 10 à 20, et présentant 6 unités de glycérine ou plus.

2. Composition d'émulsion huile dans eau contenant un caroténoïde selon la revendication 1, dans laquelle le caroténoïde est du lycopène.

3. Composition d'émulsion huile dans eau contenant un caroténoïde selon la revendication 1 ou 2, comprenant l'acide gras supérieur présentant un nombre total de carbone supérieur ou égal à 12 en une quantité allant de 0,01 fois à 0,5 fois quant au rapport de masse par rapport au caroténoïde.

4. Composition d'émulsion huile dans eau contenant un caroténoïde selon l'une quelconque des revendications 1 à 3, dans laquelle le diamètre de particules moyen de particules d'émulsion est inférieur ou égal à 100 nm.

5. Composition d'émulsion huile dans eau contenant un caroténoïde selon l'une quelconque des revendications 1 à 4, dans laquelle l'acide gras supérieur présentant un nombre total de carbone supérieur ou égal à 12 est un acide gras supérieur présentant un nombre total de carbone allant de 18 à 22.

6. Composition d'émulsion huile dans eau contenant un caroténoïde selon l'une quelconque des revendications 1 à 5, dans laquelle une teneur totale d'un agent antiseptique et d'un conservateur est inférieure ou égale à 0,001 % par rapport à la masse totale de la composition d'émulsion.

7. Procédé de production d'une composition d'émulsion, le procédé comprenant les étapes consistant à :

préparer la composition d'émulsion huile dans eau contenant un caroténoïde selon l'une quelconque des revendications 1 à 6,
puis chauffer la composition d'émulsion à une température supérieure ou égale à 70 °C immédiatement avant ou après remplissage d'un récipient de stockage avec la composition d'émulsion, et sceller le récipient de stockage ayant été rempli avec la composition d'émulsion chauffée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008013751 A
- JP H10120933 A
- JP H09157159 A
- JP 2011241177 A
- JP 2010155815 A

### Non-patent literature cited in the description

- **KAJIMOTO, DAISAN SHOBO.** *Kosanka-zai no Riron to Jissai (Theory and Practice of Antioxidants),* 1984
- **SARUWATARI, NISHINO ; TABATA, TAISEISHA.** Sanka Boshizai Handobuku (Handbook of Oxidation Inhibitors). 1976